# EUROPEAN PATENT APPLICATION

(11) **EP 3 660 245 A1**
(43) Date of publication of application: **03.06.2020**
(21) Application number: 17919588.8
(22) Date of filing: 28.07.2017
(51) Int. Cl.: E05B 1/00, A61L 2/10

(54) **ANTIBACTERIAL DOOR KNOB**

(71) Applicant: Gargash, Abdul Jabbar, Dubai (AE)
(72) Inventor: SOKOLOV, Yuriy Borisovich, Fryazino Moscow region 141195 (RU); GARGASH, Abdul Jabbar, Fryazino Moscow region 141195 (AE)
(74) Representative: Kromanis, Artis
(86) International application number: PCT/IB2017/001011
(87) International publication number: WO 2019/021031

(57) **Abstract**

The invention relates to the field of medicine, and in particular to devices for sterilizing objects with radiation, and is intended for disinfection with the diffuse ultraviolet irradiation of the door handles of public organizations, including hospitals, clinics, public catering establishments. The algorithm of work is such that allows ultraviolet radiation for disinfection in a short period of time after passage of a person. Antibacterial door handle comprises a light guide (2); UV LEDs (1) installed at the end surface of the light guide (2), an optical reflector (4), a circuit incorporating LEDs comprising a door sensor, a power source, a delay circuit and a pulse generation power LEDs. The light emitting surface of the light guide (2) is provided with points of optical inhomogeneity which are centres of dispersion of UV radiation, with the help of which diffuse irradiation of the tube surfaces is formed, and switching circuit provides the inclusion of UV LEDs (1) for a certain period of time with a delay enable the inclusion of a door opening sensor, excluding or minimizing the irradiation time of a person's hand opening the door.

## Description

### Technical Field

The invention relates to the medical devices, particularly, to the devices sterilizing objects by means of radiation and intended for decontamination of door knobs using ultraviolet radiation.

### Background

In a number of public places, multiple persons, including those to be carriers of any disease, use the same door knob, thus making microbes carry over from one person to another one. There are particular natural microbe protection methods according to which a door knob is to be made of silver- or copper-coated materials but those methods are rather expensive and, later on, a door knob surface looks dirty due to an oxidizing effect. There is a power drive (e.g. a pedal drive) door opening method, as well as methods applicable to automatic door opening mechanisms activated when a person approaches the door.

There are agents used for decontaminating door knobs using sprays or antibacterial liquids (FR 2848590, E05B1 / 00, published on 16.12.2002; UK 2382987, A61L2 / 18, published on 18.06.2003; US 5314668, A61L 2/00, published on 24.05.1994), as well as using UV lights (RU2014119403, E04F 13/074, published on 20.11.2015), but it shall be noted that UV lights being emitted in particular doses could be hazardous to human health.

The invention is a door knob design wherein decontamination effect is implemented by diffuse-type ultraviolet radiation, which allows decontamination of entire knob surface without using any direct radiation, thus being harmless for the human health. Besides, the UV radiation algorithm is such that it is actuated for a short period of time after a person leaves an active area.

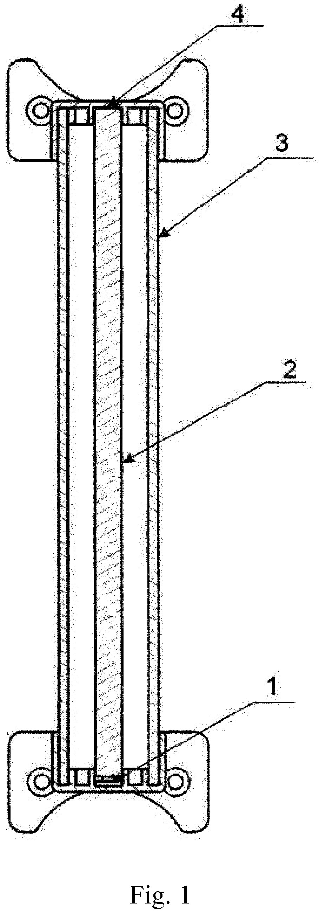

Fig. 1 indicates a variant of a door knob with LEDs 1 fitted to an end of an UV spectrum transparent light guide 2 designed as a core inserted in a tube of the same material, wherein the surface of the core is coated (using laser, printer, etc.) with inhomogeneous optical elements in the form of dots (not shown), which function as UV light diffusion (reflection) centres. While passing the optical material of light guide 2, the LED 1 emitted beam is fully reflected from the light guide walls and dissipated at inhomogeneous optical dots, wherein some portion of light goes out of the light guide. The dots are regularly arranged on the light guide 2 surface so that the entire external surface of the door knob tube is steadily irradiated by UV radiation. An air gap is maintained between the light guide 2 core and an internal wall of the knob tube 3, and on another end of the light guide 2 a reflector 4 is fitted as a reflecting surface (made of white material or alanod, alafol, etc.).

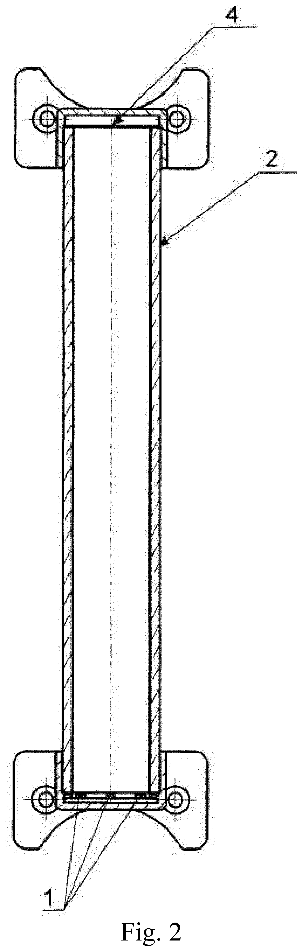

Another variant of the safe door knob is shown in Fig. 2. In this case, it is a tube 2 of the door knob that is used as a light guide and UV LEDs 1 are fitted around the tube end. An internal or external surface of the tube is coated with inhomogeneous optical dots (not shown), wherein the opposite end of the tube is fitted with a reflector 4.

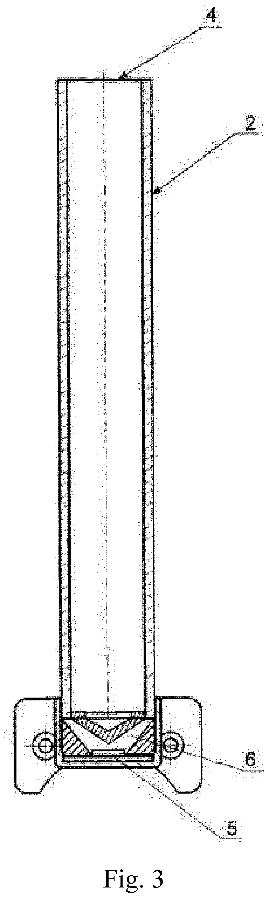

Another variant of the door knob design implements the UV COB (Chip-On-Board) and is shown in Fig. 3. In this case, a tapered former 6 of an optical beam, that corresponds to the configuration of the end of the tube 2, is fitted in front of the COB 5. The reflector 4 is fitted on the opposite end of the tube.

UV guide lights may be fitted at the outlet guide light end for improving emission power.

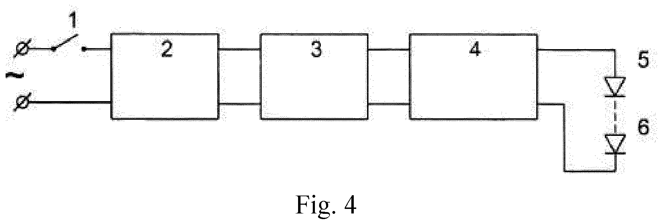

To keep any following visitor off the door knob coverage area till UV radiation is deactivated, a visible light LED, e.g. blue (Fig. 4) is to be activated along with a visitor, thereby informing that the knob is sterilized and the system is operable.

LED activation circuit is shown in Fig. 4, where:
1 - a door opening detector,
2 - a power source
3 - a LED activation delay circuit,
4 - a light pulse forming circuit,
5 - UV LEDs,
6 - visible light LEDs (e.g. blue).

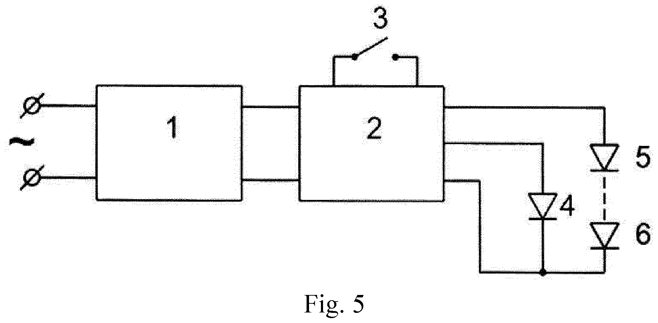

Such antibacterial door knob (Fig. 5) operates like traffic light unit. This knob is fitted with green, UV and red LEDs. Green LEDs go on (steady or flashing) in the normal state and go off several seconds later after opening a door, while UV and red LEDs light on to indicate that knob decontamination process is initiated. With the process being up, green LEDs light on again indicating that bacterial treatment is terminated.

For removing any human health UV effect, the UV LED goes on in some time after a door opening detector is activated with the glow acting for several seconds. Later, this cycle is repeated after the door is opened again.

Fig. 5 shows the traffic light LED activation circuit, where:
1 - the power source,
2 - the UV and red LED activation pulse former,
3 - the door opening detector,
4 - the green LED,
5 - the UV LED,
6 - the red LED.

End LEDs provide not only steady knob-covering diffuse radiation but this design is suitable for making the knob of aesthetic transparent material, wherein the LEDs are concealed in the knob holders and the knob glows steadily.

## Claims

1. An anti-bacterial door knob comprising a transparent tube fitted in two holders; UV LEDs; an optical reflector; a LED activation circuit including a door-opening detector, a power source, LED power pulse delay and forming circuits **characterized in that** the transparent tube is a light guide, the UV LED is positioned at one end of the light guide, a reflector is fitted on the other end of the light guide, wherein an internal surface of the light guide is coated by dots configured as a diffusion centres, by means of which the tube surface is exposed to diffuse radiation, wherein the LED activation circuit activates UV LEDs for a particular period of time to be delayed by the door opening detector, thereby eliminating or minimizing door knob-holding human hand coverage time.

2. The antibacterial door knob as per claim 1, wherein visible light LEDs (e.g. blue) are used in series along with the UV LEDs for indicating that the UV LEDs are activated.

3. The antibacterial door knob, wherein the light guide is a transparent optical core which is inserted in the tube centre with the UV LED and other LEDs are fitted on its one end and the reflector is fitted on its other end, wherein the external surface of the core is coated with dots for providing diffuse illumination to the surfaces of the knob tube, in which the light guide core is inserted.

4. The antibacterial door knob as per claim 1 operating as a traffic light unit, wherein the knob is fitted with green, UV and red LEDs, wherein the green LEDs go on (steady or flashing) in the normal state and go off several seconds later after opening a door and activation of the door-opening detector, while the UV and the red LEDs light on to indicate that the knob radiation process is initiated, wherein after completing the radiation process of the knob, the green LEDs light on signalizing that the radiation process is completed.

5. The antibacterial door knob as per claim 1, wherein an optical path former is fitted between Chip-on-Board LEDs (COB, Chip-on-Board) and light guide, wherein the form of the optical path former corresponds to the form of the end of the light guide.

6. The antibacterial door knob as per claims 1 and 3, wherein LEDs are fitted on the opposite end of the light guide like those fitted on the first end of the light guide increasing radiation capacity.
